**Europäisches Patentamt**

**European Patent Office**

**Office européen des brevets**

(19)

(11) Numéro de publication: **0 091 853**
**B1**

(12) # FASCICULE DE BREVET EUROPEEN

(45) Date de publication du fascicule du brevet:
06.08.86

(51) Int. Cl.⁴: **A 61 N 1/32**

(21) Numéro de dépôt: **83400642.1**

(22) Date de dépôt: **28.03.83**

(54) **Procédé de traitement de la peau et dispositif comportant application de ce procédé.**

(30) Priorité: **02.04.82 FR 8205796**

(43) Date de publication de la demande:
**19.10.83 Bulletin 83/42**

(45) Mention de la délivrance du brevet:
**06.08.86 Bulletin 86/32**

(84) Etats contractants désignés:
**AT BE CH DE GB IT LI LU NL SE**

(56) Documents cités:
**BE - A - 701 240**
**FR - A - 839 556**
**FR - A - 2 430 225**
**US - A - 3 897 789**

(73) Titulaire: **Simonin, Philippe, 30, Allée Horace Vernet,
F-78170 La Celle St Cloud (FR)**

(72) Inventeur: **Simonin, Philippe, 30, Allée Horace Vernet,
F-78170 La Celle St Cloud (FR)**

(74) Mandataire: **Durand, Yves Armand Louis et al, Cabinet Z.
Weinstein 20, Avenue de Friedland, F-75008 Paris (FR)**

Il est rappelé que: Dans un délai de neuf mois à compter de la date de publication de la mention de la délivrance du brevet euro-péen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne essentiellement le traitement esthétique de la peau.

On connaît déjà divers procédés et moyens de traitement ou de rajeunissement de la peau qui permettent l'effacement au moins temporaire des rides, plissements ou analogues apparaissant naturellement sur le corps humain, et notamment sur le visage, lors du vieillissement de la peau.

C'est ainsi qu'il est déjà connu de rajeunir la peau du visage notamment par application externe ou topique de certains produits cosmétiques se présentant par exemple sous la forme de crèmes ou d'onguents. Il est également connu d'effacer les rides ou irrégularités diverses apparaissant sur l'épiderme par des moyens mécaniques, c'est-à-dire, par exemple par meulage ou employ de moyens abrasifs divers.

Mais tous ces moyens de traitement de la peau par voie externe présentent un certain nombre d'inconvénients. Bien qu'ils permettent l'aplanissement et donc le rajeunissement de la peau dans une certaine mesure, ils demeurent malgré tout coûteux, car ils exigent l'intervention d'un matériel ou de produits spéciaux. D'un autre côté, ils ne permettent pas un traitement précis et localisé de la ride ou de la zone à traiter, et sont susceptibles d'engendrer des brûlures ou dommages divers qui risquent d'altérer la peau, tant en ce qui concerne les zones qu'il convient de traiter que celles qui n'ont pas besoin de l'être.

On sait également traiter les rides par injection dans le derme d'extraits placentaires ou de silicone. De telles injections permettent évidemment de regonfler les rides ou les plissements à la surface de l'épiderme, mais elles présentent aussi des inconvénients, car les produits que l'on injecte demeurent nécéssairement coûteux et il y a évidemment un risque de saignement.

La présente invention a pour but de remédier à tous les inconvénients ci-dessus ainsi qu'à d'autres en proposant un nouveau procédé qui n'exige l'utilisation d'aucun produit spécial, est peu coûteux, et présente toutes les qualités voulues d'efficacité et de résultat pour atténuer considérablement les rides et embellir la peau du sujet traité.

A cet effet, l'invention a pour objet un procédé d'utilisation d'un générateur d'impulsions auquel est raccordée une aiguille fine en vue du traitement esthétique de la peau pour en éliminer les rides, plissements ou analogues caractérisé en ce qu'il consiste à introduire ladite aiguille immédiatement en dessous de la peau et sensiblement parallélement à la ride ou au plissement à éliminer, et à faire passer dans cette aiguille un faible courant sous une fréquence basse et comprise entre environ 100 et 500 Hertz pendant un temps suffisant pour ainsi combler la dépression du plissement ou de la ride.

On notera que le document US-A 3 897 789 décrit déjà un générateur d'impulsions auquel est raccordée une aiguille fine d'un type susceptible d'être utilisé pour la mise en oeuvre du procédé selon l'invention, mais seule son utilisation pour l'acuponcture s'y trouve divulguée.

On comprend donc déjà que, contrairement aux procédés antérieurs qui préconisaient soit un traitement externe, soit une injection profonde dans le derme d'un produit spécial, on effectue, selon l'invention, une simple piqûre «à fleur de peau», sans aucun risque de saignement ni d'altération, quelconque de la peau. Une telle piqûre a une action locale et très précise sur la ride que l'on veut traiter, par le fait que le faible courant de fréquence appropriée passant dans l'aiguille réactivera très localement les tissus de façon à combler de façon durable le vide ou la dépression de la ride.

Et le demandeur a remarqué que c'est essentiellement dans le domaine des basses fréquences que l'on obtient des résultats surprenants et durables d'aplanissement des rides et d'embellissement de la peau.

On ajoutera ici que le procédé selon un mode de réalisation particulier de l'invention est encore caractérisé en ce qu'on fait passer le courant dans l'aiguille suivant des intervalles de temps compris entre environ une et quelques secondes en fonction de la nature de la zone à traiter.

On remarquera ici qu'étant donné l'extrêmement faible profondeur de la piqûre et le très faible courant passant dans l'aiguille, aucune douleur particulière ne sera ressentie par la personne traitée.

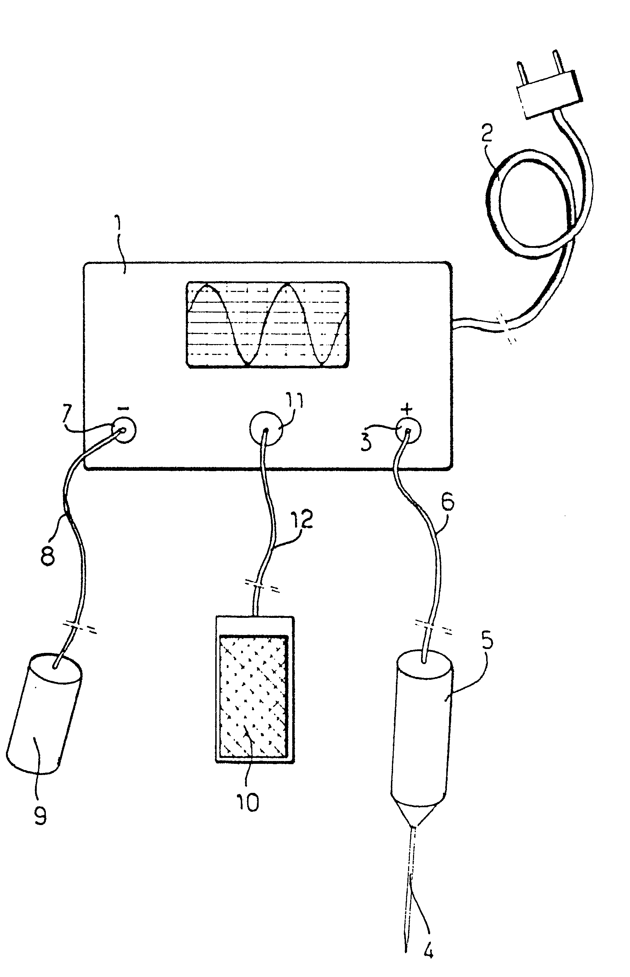

Mais d'autres caractéristiques et avantages de l'invention apparaîtront mieux dans la description détaillée qui suit et se réfère au dessin annexé dont la figure unique est une vue schématique de l'appareillage conforme aux principes de l'invention.

Suivant un exemple de réalisation, et en se reportant à la figure unique annexée, on voit qu'un dispositif susceptible d'être utilisé pour la mise en oeuvre du procédé conforme à l'invention comprend essentiellement un générateur d'impulsions 1 raccordé au réseau par une filerie 2 et à la sortie positive 3 duquel est raccordée une aiguille très fine 4. Plus précisément, l'aiguille 4 est raccordée à la sortie positive 3 du générateur 1 par un câble souple 6 et est montée de manière amovible, par tout moyen approprié, à l'extrémité d'un élément ou manchon 5 permettant la préhension par l'utilisateur.

A la sortie négative 7 du générateur 1 est raccordée, par l'intermédiaire d'un câble 8, une électrode 9 de retour du courant, cette électrode présentant par exemple une forme simplement cylindrique et étant destinée à être tenue à la main par la personne traitée.

On a monté schématiquement en 10 une pédale raccordée en 11 au générateur 1 par l'intermédiaire d'une filerie 12. Cette pédale 10, qui peut être commandée au pied, constitue un interrupteur permettant à volonté de faire passer le courant dans l'aiguille 4 suivant des intervalles de temps qui seront par exemple compris entre environ une et quelques secondes, en fonction de la nature du tissu à traiter, comme on l'expliquera en détail plus loin.

Les moyens constituant le générateur 1 n'ont pas besoin d'être décrits en détail ici. On en dira seulement qu'il comprend de préférence un circuit redresseur de la fréquence du réseau, et cela de sorte que l'aiguille 4 puisse être parcourue par un courant d'intensité très faible et de fréquence basse et comprise entre environ 100 et 500 Hertz, ce qui confère au traitement une efficacité maximum. Le générateur 1 peut bien sûr comprendre tous les éléments nécessaires pour faire varier la fréquence et l'intensité du courant à la sortie 3 du générateur, en fonction de tissus que l'on veut traiter et des résultats que l'on désire obtenir. C'est ainsi qu'on pourra utiliser une fréquence de 100 Hertz pour traiter tel endroit particulier du visage et une autre fréquence bien définie et supérieure à 100 Hertz, par exemple 400 Hertz pour traiter telle autre partie du visage.

On décrira maintenant le fonctionnement et l'utilisation de l'appareillage ci-dessus.

Le générateur 1 ayant été mis sous tension et la personne à traiter tenant en main l'électrode 9, l'opérateur saisit l'aiguille 4 par son support 5 et introduit cette aiguille juste sous la peau, c'est-à-dire immédiatement en dessous de la ride à traiter, ce qui ne procure aucune douleur ni saignement, étant donné la profondeur extrémement faible de pénétration de l'aiguille. Il est essentiel que la piqûre soit effectuée sensiblement parallèlement à la surface de l'épiderme, c'est-à-dire sensiblement parallèlement à la ride traitée. La piqûre étant faite, l'opérateur, en agissant sur la pédale 10 fera passer dans l'aiguille 4 un courant de faible intensité et de fréquence 100 Hertz par exemple étant bien entendu encore une fois, que le générateur 1 peut être prévu pour faire varier la fréquence et l'établir à une valeur supérieure, en fonction de la nature de la ride à traiter et des résultats recherchés. De même, grâce à la pédale 10, on pourra faire varier le nombre d'impulsions et le temps de passage du courant en fonction de la nature des rides à traiter.

Ainsi, le passage du courant provoquera dans les tissus une réactivation du métabolisme cellulaire, ou encore une petite réaction inflammatoire produisant un exsudat apte à combler le vide ou la dépression de la ride qui s'aplanira. Bien entendu, on pourra répéter l'opération si au bout de quelques temps la ride se creusait à nouveau ou réapparaissat, mais à la longue la ride se trouve très fortement atténuée, et cela de façon durable, en raison de la meilleure vitalité tissulaire créée sous la ride après plusieurs excitations résultant du courant de faible intensité et basse fréquence passant dans l'aiguille.

Ainsi le demandeur a constaté que pour une fréquence de 400 Hertz on obtenait une atténuation durable de 50 à 80% des rides au niveau des lèvres, et ce au bout de cinq séances de traitement. De même à 100 Hertz, pour les rides au niveau des yeux et des cernes, on obtient une atténuation durable de 30 à 50% desdites rides, à raison d'environ 5% par séance et ce de façon cumulative.

Bien entendu, l'aiguille fine 4 peut présenter en section des formes et des dimensions diverses en fonction des tissus à traiter, le seul critère étant que cette aiguille demeure relativement fine pour pouvoir pénétrer dans la peau très près de sa surface sans risque de l'abîmer au d'y créer des fissures ou des déchirements, ce qu'il convient à tout prix d'éviter comme on le comprend. De même, les moyens de fixation amovibles de l'aiguille 4 sur la partie 5, ainsi que les divers constituants du générateur 1 peuvent être quelconques et appropriés.

On a donc réalisé suivant l'invention un procédé de traitement des rides par un faible courant sous basse fréquence «injecté» par piqûre immédiatement en dessous des rides ou plissements à traiter, parallèlement à ceux-ci, et permettant d'embellir la peau de façon persistante sans aucun risque de la détériorer et sans aucune douleur pour la personne subissant le traitement.

On ajoutera ici que les rides constituant non pas une maladie mais un phénomène normal et naturel qui apparaît lors du vieillissement de la peau, le procédé de l'invention ne peut en aucun cas être considéré comme une méthode thérapeutique ou curative. Et on insistera ici sur le fait que l'aiguille introduite immédiatement sous la surface de la peau parallèlement à la ride ne provoque aucun saignement et ne sert à introduire aucun produit dans la peau, ce qui serait caractéristique d'un acte médical. D'ailleurs le procédé de l'invention peut être parfaitement et légalement exécuté par une personne qui n'est pas médecin, telle que par exemple un esthéticien.

Bien entendu, le procédé de traitement de la peau selon l'invention ne doit pas être considéré comme s'appliquant limitativement au traitement des rides. En effet ils peut, d'une manière générale, s'appliquer au traitement de toutes les irrégularités de la peau, telles que par exemple les cicatrices ou les vergetures, et cela sans sortir du cadre de l'invention.

**Revendications**

1. Procédé d'utilisation d'un générateur d'impulsions (1) auquel est raccordée une aiguille fine (4) en vue du traitement esthétique de la peau pour éliminer les rides, plissements ou analogues, caractérisé en ce qu'il consiste à introduire ladite aiguille immédiatement en dessous de la peau et sensiblement parallèlement à la ride ou au plissement à éliminer, et à faire passer dans cette aiguille un courant sous une fréquence basse et comprise entre 100 et 500 Hertz pendant un temps suffisant pour combler la dépression du plissement ou de la ride.

2. Procédé selon la revendication 1, caractérisé en ce qu'on fait passer le courant dans l'aiguille (4) suivant des intervalles de temps compris chacun entre une et quelques secondes.

3. Procédé selon la revendication 1 ou 2, caractérisé en ce qu'on utilise un courant de fréquence égale à 100 Hertz.

4. Procédé selon la revendication 1 ou 2, caractérisé en ce qu'on utilise un courant de fréquence égale à 400 Hertz.

## Patentansprüche

1. Verfahren zur Verwendung eines Impulsgenerators (1) an welchen eine feine Nadel (4) zwecks ästhetischer Behandlung der Haut angeschlossen ist, um die Runzeln, Falten oder dgl. zu entfernen, dadurch gekennzeichnet, dass es darin besteht, die besagte Nadel unmittelbar unter die Haut und im wesentlichen parallel zu der zu entfernenden Runzel bzw. Falte einzuführen und in diese Nadel einen Strom bei einer zwischen 100 und 500 Hertz liegenden Niederfrequenz während einer zum Ausfüllen der Vertiefung der Falte bzw. der Runzel ausreichenden Zeit hindurchzulassen.

2. Verfahren nach Anspruch 1, dadurch gekennzeichnet, dass man den Strom in jeweils zwischen einer und einigen Sekunden liegenden Zeitspannen durch die Nadel (4) hindurchlässt.

3. Verfahren nach Anspruch 1 oder 2, dadurch gekennzeichnet, dass man einen Strom mit einer 100 Hertz betragenden Frequenz verwendet.

4. Verfahren nach Anspruch 1 oder 2, dadurch gekennzeichnet, dass man einen Strom mit einer 400 Hertz betragenden Frequenz verwendet.

## Claims

1. Method of using a pulse generator (1) to which is connected a fine needle (4) for the aesthetic treatment of the skin for removing wrinkles, puckerings or the like, characterized in that it consists in introducing the said needle immediately underneath the skin and in substantially parallel relation to the wrinkle or to the puckering to be removed and in passing into this needle a current at a low frequency comprised between 100 and 500 Hertz for a time sufficient to fill up the depression of the puckering or of the wrinkle.

2. Method according to claim 1, characterized in that one passes the current into the needle (4) according to time intervals comprised each one between one and several seconds.

3. Method according to claim 1 or 2, characterized in that one uses a current of a frequency equal to 100 Hertz.

4. Method according to claim 1 or 2, characterized in that one uses a current of a frequency equal to 400 Hertz.